# EUROPEAN PATENT APPLICATION

(11) **EP 1 577 802 A1**
(43) Date of publication of application: **21.09.2005**
(21) Application number: 04026273.5
(22) Date of filing: 05.11.2004
(51) Int. Cl.: G06F 17/50

(54) **Computer program for designing a microfluidic device**

(30) Priority: 27.02.2004 JP 2004055354
(71) Applicant: Hitachi, Ltd., Tokyo (JP)
(72) Inventor: Miyake, Ryo Hitachi, Ltd., Intell. Property Group, Chiyoda-ku Tokyo 100-8220 (JP); Otaguro, Toshio Hitachi, Ltd., Intell. Prop. Group, Chiyoda-ku Tokyo 100-8220 (JP); Okada, Ryoji Hitachi, Ltd., Intell. Property Group, Chiyoda-ku Tokyo 100-8220 (JP); Sasaki, Naoya Hitachi, Ltd., Int. Property Group, Chiyoda-ku Tokyo 100-8220 (JP)
(74) Representative: Beetz & Partner

(57) **Abstract**

The present invention relates to a computer program embodied on a computer readable medium for designing a treating member (10). The computer program contains a first code segment adapted to acquiring a piece of input information containing a treatment sequence (ST1); and a second code segment adapted to designing a flow path along which a sample moves, based on the piece of input information (ST2).

## Description

### Background of the Invention

The present invention relates to a program for designing a treating member to be used when treatments such as chemical reactions are conducted as an automated process, and a system for providing a service to design a treating member using the program concerned.

In general, a chemical treatment process is constituted with a sequence of individual treatments referred to as unit chemical operations such as centrifugal separation and mixing/reaction. Various processes have been developed, and these various chemical treatment processes require complicated operations; generally, individual treatment steps are carried out by hand sequentially in such a way that samples and reagents are placed in glass flasks and test tubes. However, for some operations, automated devices are available. Examples of such automated devices include a centrifugal separator in which components in a sample solution inside a test tube are separated by specific gravity by revolving the test tube, an evaporator device in which a flask is made to rotate and the liquid inside the flask is evaporated, and a general-purpose robot device which sucks up a trace of liquid inside a test tube and transfers the liquid to another test tube or a reaction vessel.

WO 00/78455 describes a microstructure and a method for examination using amplification as a device automating by rotary drive a particular chemical treatment process and a particular chemical analysis, both having been predetermined. In this device, by use of a method for purifying and separating a nucleic acid mixture, described in JP 8-501321 A, a DNA mixture solution is made to pass through a glass filter as an inorganic substrate, and thereafter made to pass through a cleaning liquid and an eluent, thus merely the DNA being recovered. The glass filter is arranged on a rotatable structure, and reagents such as the cleaning liquid and the eluent are held respectively in individual reagent reservoirs in the structure. The respective regents are made to flow by the centrifugal force generated by the rotation of the structure, and the valves arranged in the micro flow paths connecting between the respective reagent reservoirs and the glass filter are opened, the reagents being thereby made to pass through the glass filter.

As can be seen clearly from the above description on conventional techniques, in a sequential chemical treatment process composed of a sequence of different unit chemical operations, the individual unit chemical operations are needed to be carried out by hand or by combining devices; the state of affairs is such that there have not yet been proposed a technique and a device which can completely automate a sequential process from beginning to end. In the aforementioned WO 00/78455, there is disclosed a technique in which for a particular treatment process of gene extraction, a sequence of operations are completely automated by making liquids flow by exerting centrifugal force through rotary driving; however, this technique cannot meet arbitrary chemical treatment processes.

Furthermore, for the purpose of carrying out an arbitrary chemical treatment process, microstructures are needed to be designed in compliance with the plural unit chemical operations constituting the chemical treatment process. However, there has not yet been known a program or a system which provides microstructures in compliance with an arbitrary chemical treatment process.

Consequently, the present invention takes as its object the provision of a program for designing a treating member which program can provide a treating member automating a sequential process in compliance with an arbitrary treatment process and a system for providing a service system to design the treating member.

### Brief Summary of the Invention

A program for designing a treating member involved in the present invention which program has achieved the aforementioned object may include a step for acquiring a piece of input information including a treatment sequence; and a step for designing a flow path, along which a sample moves, based on the above described piece of input information. According to the present program, there can be constructed a piece of drive control information about the designed treating member.

On the other hand, the system for designing a treating member involved in the present invention may include a step for acquiring a piece of design information about a flow path along which a sample moves, designed based on a piece of input information including a treatment sequence; a step for creating a piece of control information for controlling a treating-member fabrication unit based on the above described piece of design information; and a step for outputting the above described piece of control information to the above described treating-member fabrication unit. In the present system, there can be constructed a piece of drive control information about the designed treating member, and there can be output the piece of drive control information to the terminal having output the piece of design information.

Additionally, the system for designing a treating member involved in the present invention may include a step for acquiring a piece of input information including a treatment sequence; a step for designing a flow path along which a sample moves based on the above described piece of input information; a step for creating a piece of control information for controlling a treating-member fabrication unit based on a piece of design information about the designed flow path; and a step for outputting the above described piece of control information to the above described treating-member fabrication unit.

It is preferable that in the program and system for designing the treating member, involved in the present invention, in addition to a piece of information about the treatment sequence, the piece of input information further include one or more pieces of information selected from the group consisting of a piece of operation information about the above described sample, a piece of treatment condition information about the above described sample, and a piece of information associated with samples. In the program and system for designing the treating member, involved in the present invention, the flow path can be designed by optimizing the piece of shape information, read out from the database in which the treatment sequence and the piece of shape information are stored to correlate with each other, based on a piece of information included in the piece of input information.

According to the present invention, there can be provided a treating member which automates a sequential process in compliance with an arbitrary chemical treatment process. More specifically, according to the program for designing a treating member and the system for designing a treating member, involved in the present invention, there can be designed a treating member for carrying out an arbitrary process, based on a piece of information about the treatment sequence included in the piece of input information.

Other objects, features and advantages of the invention will become apparent from the following description of the embodiments of the invention taken in conjunction with the accompanying drawings.

### Brief Description of the Several Views of the Drawing

Fig. 1 is a block diagram showing a system for designing a treating member involved in the present invention;
Fig. 2 is a block diagram of a device for carrying out a chemical treatment process;
Fig. 3 is a block diagram of a device for carrying out a chemical treatment process using a disc-shaped treating member;
Fig. 4 is a flowchart for a fabrication method of a treating member, applied to the program for designing a treating member involved in the present invention;
Fig. 5 is a flowchart for illustrating the information flow in a system for designing a treating member;
Fig. 6 is an illustrative diagram for an algorithm in the program for designing a treating member; and
Fig. 7 is a flowchart showing steps for fabricating a treating member based on a piece of design information about the treating member.

### Detailed Description of the Invention

Detailed description will be made below on the program for designing the treating member and the system for designing the treating member, both involved in the present invention, with reference to the accompanying drawings.

### <First best embodiment>

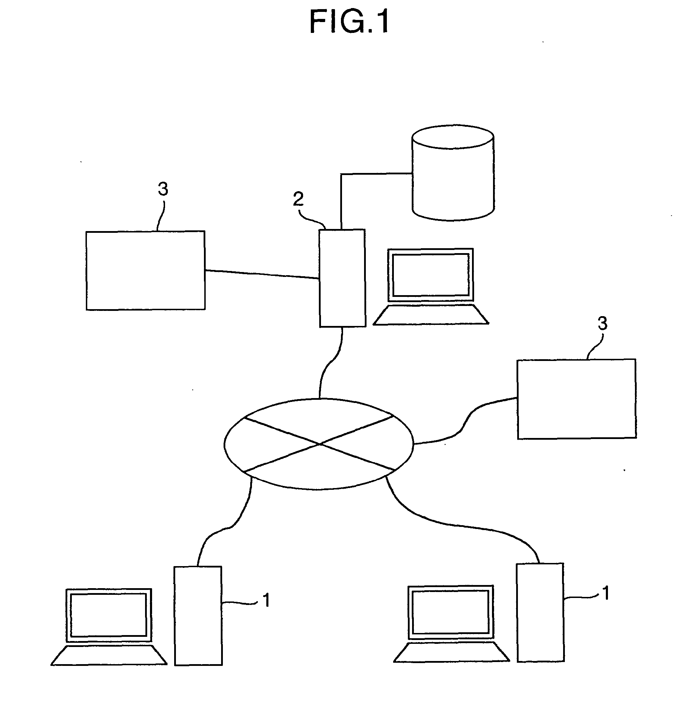

As shown in Fig. 1, the system for designing the treating member according to the present invention includes a user terminal 1 belonging to the system user side, a provision terminal 2 for designing a flow path in the treating member to be used in a chemical or other treatment process, and a treating-member fabrication unit 3 for fabricating the treating member. In the present system, the user terminal 1 and the provision terminal 2 are connected to each other through the intermediary of communication network such as Internet and intranet, and can exchange various types of data with each other. Incidentally, in the present system, the unit 3 for fabricating the treating member may be directly connected to the provision terminal 2, or may be connected to the provision terminal 2 through the intermediary of communication networks in a communicatable manner.

In the present system, a treating member to actualize the treatment sequence desired by a user of the system is designed on the provision terminal 2, and the treating member is fabricated by the unit 3 for fabricating the treating member based on this design. More specifically, on the provision terminal 2, according to a processing program to be described later in detail, there are executed a step for acquiring a piece of input information including the treatment sequence desired by the user of the system and a step for designing the flow path in the treating member based on the above described piece of input information.

Firstly, a chemical treatment process will be described as a treatment process in which a treating member fabricated by taking advantage of the present system for designing a treating member. Incidentally, the treatment process using the treating member is not limited to a chemical treatment process, but may be a treatment process such as a process in which merely a physical treatment is carried out.

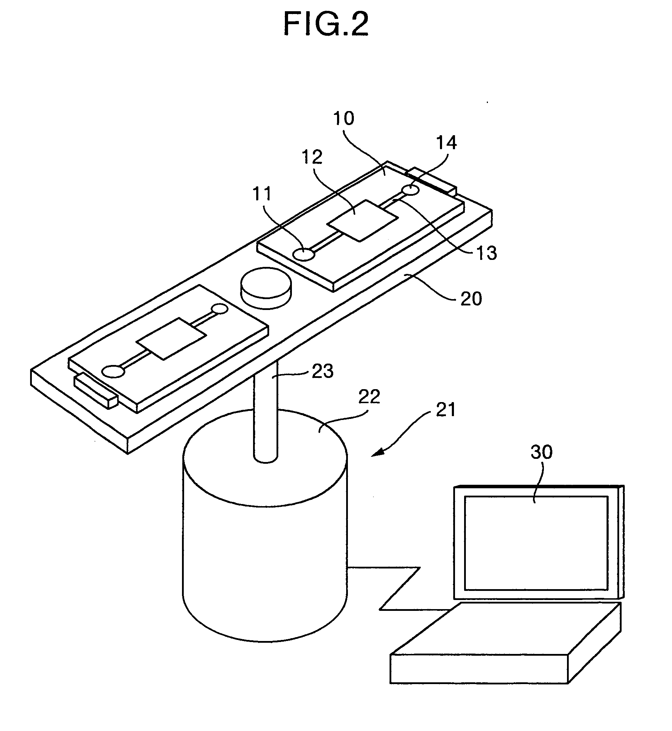

As shown in Fig. 2, a device for carrying out a chemical treatment process includes a fixing jig 20 for placing and holding a treating member 10 on the top thereof, a rotary drive mechanism 21 to turn the fixing jig 20, a control section 30 to control the turning of the fixing jig 20 by the rotary drive mechanism 21. Into the control section 30, for example, the rotational velocity, time of rotation, stop time and the like of the fixing jig 20 are input, and the control section 30 controls the rotary drive mechanism 21 according to this piece of input information. The rotary drive mechanism 21 includes a drive member 22 such as a stepping motor and a shaft 23 connected to the drive member 22. The fixing jig 20 is fixed to the shaft 23 of the rotary drive mechanism 21. On the fixing jig 20, a pair of treating members 10 can be disposed symmetrically with the shaft 23 of the rotary drive mechanism 21 as a center therebetween.

The treating member 10 is arranged with an inlet 11 for introducing a sample in the vicinity of one end thereof, and an outlet 14 for recovering the sample in the vicinity of the other end thereof. The treating member 10 has a flow path consisting of a groove 13 along which a sample flows and a reaction section 12 to apply a predetermined treatment to the sample, between the inlet 11 and the outlet 14. The treating member 10 like this can be fabricated by molding a resin such as polypropylene.

The treating member 10 is temporarily fixed onto the fixing jig 20 in such a way that the end in the vicinity of which the inlet 11 is arranged is located on the side of the shaft 23 of the rotary drive mechanism 21 and the other end in the vicinity of which the outlet 14 is arranged is located to be away from the shaft 23.

The device for carrying out a chemical treatment process configured as described above is operated as follows. Firstly, an operator drops a sample into the inlet 11 of the treating member 10, and thereafter the treating member 10 is fixed onto the fixing jig 20. Additionally, the operator inputs predetermined treatment conditions (for example, the rotation velocity, rotation time and stop time) into the control section 30 for the purpose of carrying out a desired chemical treatment process.

Thereafter, according to the input procedures, the rotary drive mechanism 21 repeats rotation and halt. When the treating member 10 rotates together with the fixing jig 20 with the aid of the action of the rotary drive mechanism 21, the sample migrates toward the outer circumference owing to centrifugal force. Additionally, when the rotation is halted, the migration of the sample is also halted. Consequently, by appropriately repeating the rotation and halt, the sample dropped into the treating member 10 can be appropriately made to migrate and halted toward the circumference. More specifically, according to the treatment conditions input by the operator, the sample is guided into the flow path consisting of the groove 13 and the reaction section 12, and thus a desired chemical treatment process can be applied. In the reaction section 12, a reaction of the sample is started. Plural reaction sections (not shown in the figure) may be arranged at positions downstream of the reaction section 12, namely, at positions on the circumference side of the reaction section 12, as the case may be. By further applying rotation and halt, the sample is successively delivered and different unit chemical operations can be sequentially carried out.

Incidentally, in the above described explanation, description is made on the case where the procedures of the chemical treatment process are input each time; however, as is obvious, a recording medium on which procedures have been beforehand recorded may be provided and operations may be carried out sequentially by reading out therefrom a piece of information about the procedures.

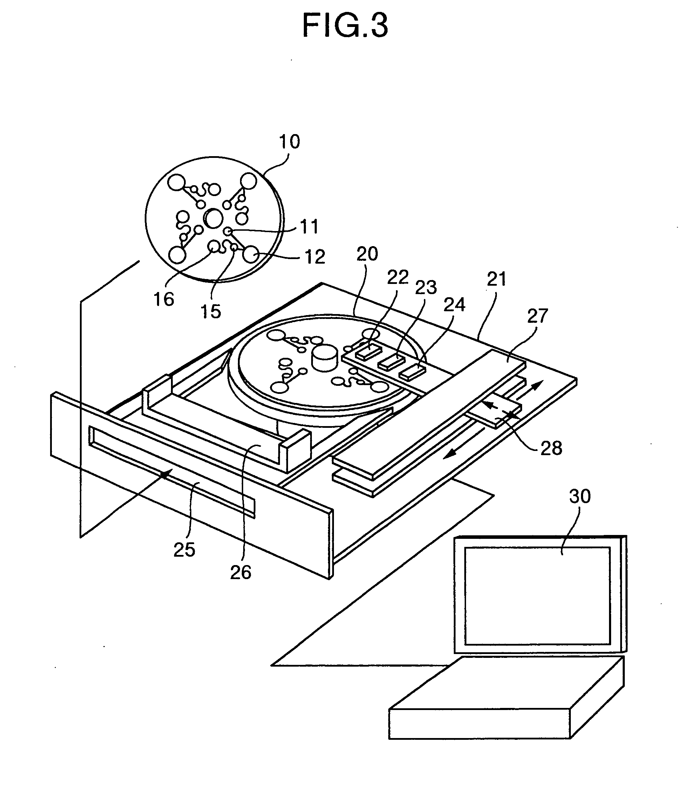

Additionally, the chemical treatment process can also be carried out by means of such a device as shown in Fig. 3. The device shown in Fig. 3 is a device in which a chemical treatment process is carried out by using a treating member 10 made of a disc.

In the treating member 10, sample introduction inlets 11 are arranged in the vicinity of the rotational center of the disc. On the disc surface of the treating member 10, flow paths each consisting of a micro reaction section 12 and the like are arranged. The treating member 10 also comprises a reagent charging section 16 which contains a reagent in advance. However, a flow path blocking section 15 made of a thermosensitive material is arranged between the reagent charging section 16 and a reaction section 12. The flow path blocking section 15 prevents the sample charged in the reagent charging section 16 from flowing into the reaction section 12. The flow path blocking section 15 has a function such that the section is fused when the section receives an amount of heat larger than a certain value, and thus the flow path is opened.

Incidentally, the treating member 10 may be covered with a case although the case is not shown in the figure. In the case where the treating member 10 is covered with a case, the surface of the case concerned is provided a slit for use in introducing therethrough a sample into the treating member 10 and for use in operating or detecting therethrough the flow of the sample.

The drive device 21 is arranged with an insertion slot 25 for inserting the treating member 10. A fixing jig 20 is arranged at a position away from the insertion slot. The fixing jig 20 is connected to a rotary mechanism 26. A sample handling device 22 for controlling the flow of the sample, a heating device 23 for heating the sample and a measurement device 24 for measuring the reaction condition of the sample are arranged on a drive arm 27. The measurement device 24 is arranged with an irradiation light source and a photodetector for detecting the intensity and spectrum of the reflected light. The drive arm 27 is connected to an arm drive mechanism 28 for scanning from the center of the circle of the treating member 10 toward the circumference thereof. The drive mechanism 21 is connected to an input unit 30. Alternatively, the drive mechanism 21 and the input unit 30 may be integrated into one unit. With the configuration described above, the operations are developed as follows.

Firstly, a sample is dropped into the introduction inlets 11 of the treating member 10. The treating member 10 is inserted into the insertion slot 25. The treating member 10 is set onto the fixing jig 20 in a manner cooperating with the insertion slot 25. The condition thus formed completes the preparation for startup.

In the next place, the operator reads out a piece of operation control information from a memory device in the input unit, corresponding to the chemical treatment process for the treating member 10 piece with the aid of the input unit 30. With a startup command, the treating member 10 starts to rotate. The sample is starts to flow toward the circumference owing to the centrifugal force accompanying the rotation. After a predetermined elapsed time, the disc decreases the rotation velocity, the treating member 10 is thereby made to rotate slowly, and the flow path blocking section 15 on the treating member 10 is adjusted in position to face the sample handling device 22.

A predetermined beam of light is irradiated from the sample handling device 22 onto the flow path blocking section 15. The flow path blocking section 15 is heated by the beam of light and fused, and the reagent charging section 16 and the reaction section 12 are made to communicatively connected with each other. When the treating member 10 starts to rotate once again from this condition, the sample in the reagent charging section 16 flows into the reaction section 12 owing to centrifugal force. Thus, the sample and a reagent can be mixed together in the reaction section 12.

The rotation velocity is once again decreased, and the above described reaction section 12 is in turn adjusted in position to face the heating device 23. The reaction section 12 is heated by the radiation heat due to a heater in the heating device 23. The reaction is promoted by this heating. After heating for a predetermined time, the arm drive mechanism 27 operates to locate the measurement device 24 directly above the above described reaction section 12. Thereafter, a predetermined beam of light is irradiated toward the reaction section 12 from the irradiation light source of the measurement device 24. The photodetector in the measurement device 24 can find the extent of reaction, the concentrations and the like of the components contributed to the reaction in the sample by detecting a set of optical information including the absorption spectrum, fluorescence or the like in the reaction section 12.

In the above described example, the flow path blocking section 15 is fused by irradiating a beam of light from the sample handling device 22 to communicate the reagent charging section 16 with the reaction section 12; however, as is obvious, the blockade may be removed by a mechanical boring operation or the like. Additionally, also in the heating device 23, heating may be made by direct contact with a heater. Alternatively, the whole treating member 10 may be heated.

In the above described Example, description has been made on the mixing operation of one sample with one reagent; however, as is obvious, many reactions can be combined stepwise by arranging plural reagents and plural reaction vessels, and plural blocking sections corresponding thereto along a direction toward the circumference. In addition to reactions by mixing, component separation can be made possible by arranging a chromatographic function on the way of the flow path. Additionally, increase of the rotation number makes it possible to give the treating member a function to separate components in the vessel based on the difference in specific gravity.

According to the above described example, for an arbitrary chemical treatment process, it comes to be possible to carry out the arbitrary chemical treatment process by using a treating member 10 consistent with the process. Additionally, in the treating member 10 placed in a case, samples and reagents are not scattered during its rotation, so that an extremely safe chemical treatment is possible.

As described above, by using a treating member 10 capable of carrying out plural different unit chemical operations, an arbitrary chemical treatment process can be carried out. In the next place, description will be made on the program for designing a treating member for use in acquiring a piece of design information about the treating member 10 on the occasion of fabricating the treating member 10 to be used in the aforementioned chemical treatment process. In the following description, as an example of the treating member 10, there is adopted a treating member for carrying out operation to separate micro particles such as bacteria present in urine, environmental water and the like.

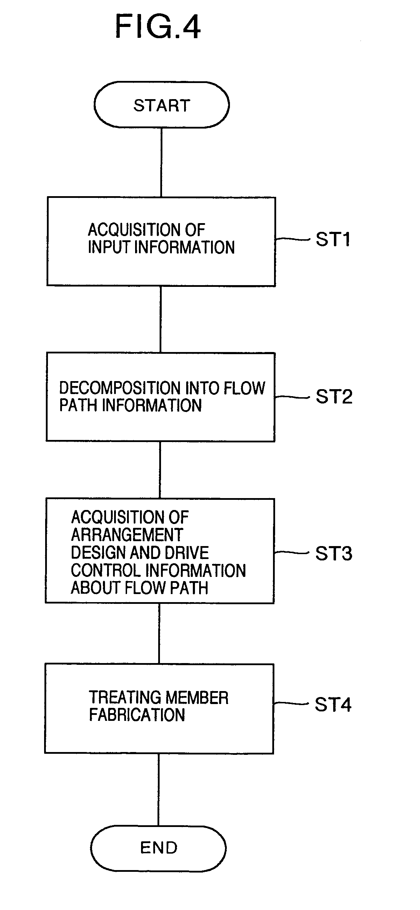

The present program for designing a treating member can design a treating member 10 on the provision terminal 2 according to the flowchart as shown in Fig. 4. In other words, the present program for designing a treating member is installed in the provision terminal, and designs on the provision terminal a treating member for carrying out the treatment sequence desired by a system user.

In the flowchart shown in Fig. 4, firstly in step 1 (represented by "ST1" in Fig. 4, and the following steps being represented similarly), a piece of input information including the treatment sequence is acquired from the user terminal 1. Here, the treatment sequence means a piece of information representing as a time series the unit chemical operations included in the chemical treatment process desired by the system user. The piece of input information includes, in addition to the piece of information representing the treatment sequence, a piece of liquid sample related information such as the liquid amount and liquid viscosity of the analyte sample used in chemical treatment process, a piece of treatment condition information, a piece of information about the apparatus owned by the system user, a piece of ID information specific to the system user and the like.

In particular, the unit chemical operations include a centrifugal separation operation, a mixing operation, a fractionation operation and the like. When the piece of information representing the treatment sequence covers a mixing operation, it is preferable that a set of information about the liquid amount and the composition of the solution to be the object of mixing is concurrently acquired as a part of the piece of input information. Additionally, when the piece of information representing the treatment sequence covers a fractionation operation, it is preferable that a piece of information about the liquid amount and the component to be fractionated of the solution is concurrently acquired as a part of the piece of input information.

Specifically, as an example, there is acquired a piece of input information covering the sequential unit chemical operations represented by the following operations (1) to (8).
(1) The solid content in the sample solution is precipitated by means of centrifugal separation.
(2) Merely the clear supernatant liquid is fractionated.
(3) A solvent to dissolve oil and fat components is added to and mixed with the clear supernatant liquid.
(4) The above described solvent is precipitated with the aid of centrifugal operation.
(5) Merely the clear supernatant liquid is fractionated.
(6) The clear supernatant liquid is subjected to stronger centrifugal operation, and micro particles such as bacteria are precipitated.
(7) The clear supernatant liquid is removed.
(8) The precipitate is recovered.

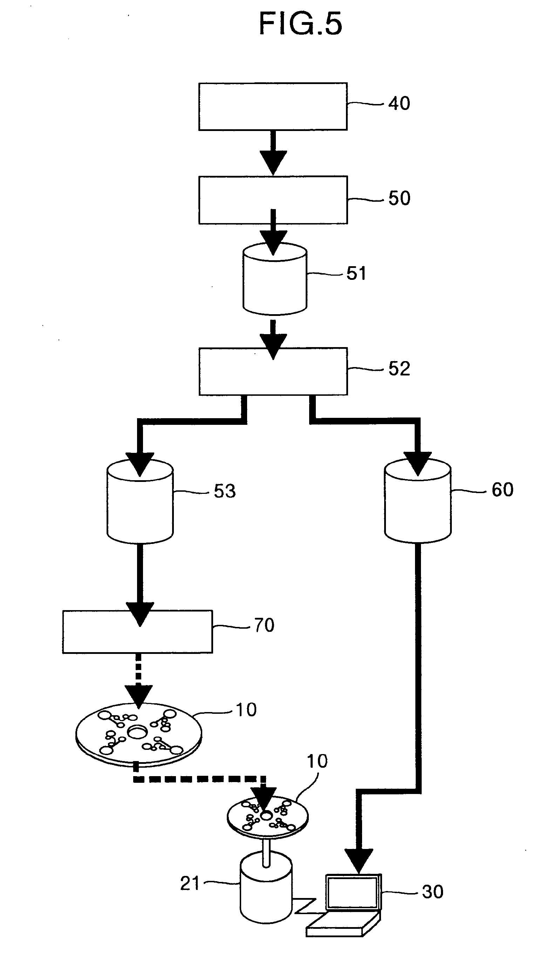

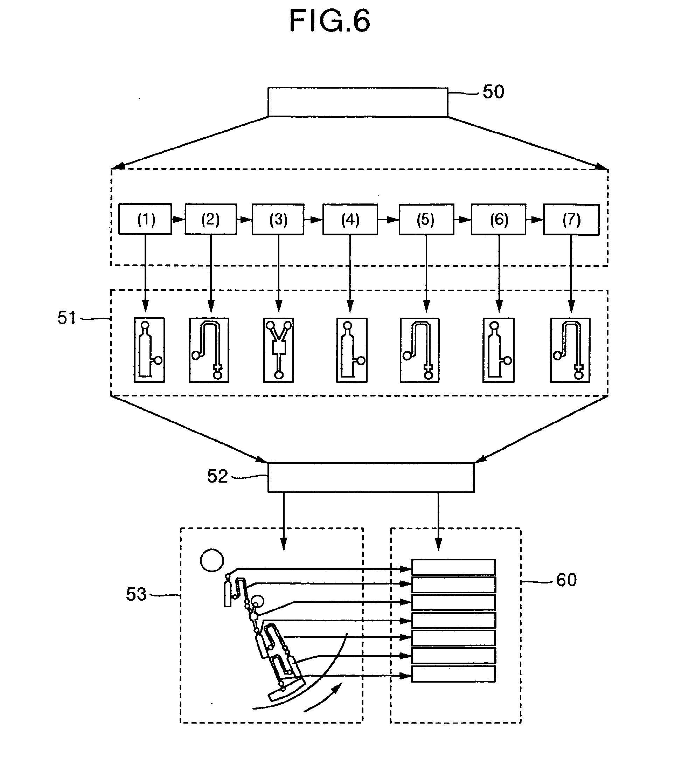

In the next place, in the step 2, the piece of input information acquired in step 1 is decomposed into pieces of information about the flow path on the rotary member, for example, with the aid of the system for designing a treating member as shown in Fig. 5 and Fig. 6. More specifically, the step 2 selects a piece of path shape/dimension information for each of the plural unit chemical operations included in the piece of input information. For example, the present program can also select a piece of path shape/dimension information in compliance with the plural unit chemical operations included in the piece of input information, by use of a database in which a piece of information about the unit chemical operations and a piece of path shape/dimension information appropriate to the unit chemical operations concerned are stored to correlate with each other.

Specifically, when a piece of input information covering the above described unit chemical operations (1) to (8) is acquired, a piece of design information about a treating member is acquired in the following way as shown in Fig. 6.

Firstly, by making a flow path designing tool 50 retrieve the database, as a piece of flow path shape/dimension information corresponding to the unit chemical operation (1), a flow path element for use in centrifugal separation extending along the centrifugal force direction (a radial direction) is given to be a fundamental shape. In the next place, there are given specific dimensions including the length and width of the flow path in compliance with the piece of sample liquid amount information included in the piece of input information, the fundamental shape is corrected on the basis thereof, and the piece of flow path shape/dimension information corresponding to the unit chemical operation (1) is acquired. The piece of flow path shape/dimension information is temporarily accumulated in a recording device 51 as a piece of flow path information for carrying out the unit chemical operation (1).

When a piece of input information acquired includes the sample composition, the physical property values of a material to be precipitated and the like, a piece of information about the acceleration to be exerted by the centrifugal separation, the time required and the like is obtained by calculation based on the piece of input information. In this case, as for the unit chemical operation (1), a piece of operation information about the device for carrying out the chemical treatment process can be obtained based on these pieces of information. The set of operation information obtained is accumulated in the recording device 51.

In the next place, by making the flow path designing tool 50 retrieve the database, as a piece of flow path shape/dimension information corresponding to the unit chemical operation (2), in the first instance, a capillary flow path element for fractionation, transversely extending from the side face of the flow path element for centrifugal separation designed in the above described (1) and bent to the upstream side, is given to be a fundamental shape. The liquid flow in the capillary flow path element for fractionation is described. As a first step, the centrifugal separation is completed, and the rotation of the rotary member is halted; then, the clear supernatant liquid in the flow path element for centrifugal separation fills the capillary flow path element for fractionation owing to the capillary force. When the rotation is started once again, because the outlet of the capillary flow path element for fractionation is situated in an outer position away from the rotation center than the inlet, the clear supernatant liquid in the flow path element for centrifugal separation starts to continuously flow owing to the siphon effect. Then, based on the piece of physical property information representing the surface tension of the sample solution and the like, included in the piece of input information, the width of the flow path and the like for the capillary flow are generated as a piece of design information, and on the generated piece of design information, the fundamental shape is corrected to acquire the piece of flow path shape/dimension information corresponding to the unit chemical operation (2). The piece of flow path shape/dimension information is temporarily accumulated in the recording device 51 as a piece of flow path information for carrying out the unit chemical operation (2).

If the surface tension coefficient, the viscosity, etc. of the clear supernatant liquid to be fractionated are acquired as a piece of input information, based on these pieces of information, the minimum time for the clear supernatant liquid to fill the capillary flow path element for fractionation and the minimum time needed for complete flow out of the clear supernatant liquid from the interior of the flow path element for centrifugal separation by means of a syphon are obtained by calculation. In this case, as for the unit chemical operation (2), a piece of operation information about the device for carrying out the chemical treatment process can be obtained based on these pieces of information. The piece of operation information obtained is accumulated in the recording device 51.

In the next place, as a piece of flow path shape/dimension information corresponding to the unit chemical operation (3), as a first step, there is given a flow path element for mixing which forms one flow path from two confluent flow paths extending along the direction of the centrifugal force (the radial direction). In the next place, there are given specific dimensions and the like based on the piece of information about the liquid amount, the viscosity, the composition and the like of the solution to be an object of mixing, included in the piece of input information, the fundamental shape is corrected on the basis thereof, and the piece of flow path shape/dimension information corresponding to the unit chemical operation (3) is acquired. The piece of flow path shape/dimension information is temporarily accumulated in the recording device 51 as a piece of flow path information for carrying out the unit chemical operation (3).

Incidentally, on the occasion of the unit chemical operation (3), there can be obtained a piece of information about the viscosities and affinities of two liquids to be mixed together based on the database. Additionally, when one liquid is an aqueous solution and the other liquid is an organic solvent, and accordingly there is needed an operation in which the two liquids are mutually, finely dispersed and emulsified, the fundamental shape of the above described flow path element for mixing can be corrected in such a way that a large number of micro protrusions or a large number of micro convexities and concavities are arranged inside the flow path element.

Additionally, based on the pieces of physical property value information about the two liquids to be mixed together, there can be obtained by calculation a piece of operation information including the flow velocities with which the two liquids should be respectively introduced into the flow path element for mixing. In this case, for the unit chemical operation (3), a piece of operation information about the device for carrying out the chemical treatment process can be obtained based on these pieces of information. The obtained piece of operation information is accumulated in the recording device 51.

As described below, similarly to the aforementioned (1) to (3), there is also given a fundamental shape to each of the unit chemical operations (4) to (7), the fundamental shape is corrected according to a piece of information included in the piece of input information; thus, there can be obtained pieces of path shape/dimension information respectively corresponding to the unit chemical operations (4) to (7). Additionally, in a similar manner, for the unit chemical operations (4) to (7), there can be acquired pieces of operation information about the device with which the chemical treatment process is carried out. Incidentally, because the unit chemical operation (8) is an operation which recovers the precipitate formed by the unit chemical operation (6), no particular piece of flow path shape/dimension information is needed.

In the above described manner, the pieces of flow path shape/dimension information for carrying out the respective unit chemical operations (1) to (7) and the pieces of operation information in the respective unit chemical operations can be sequentially accumulated in the recording device 51.

In the next place, in step 3, the pieces of flow path shape/dimension information, accumulated in the recording device 51, for carrying out the respective unit chemical operations are read out, and the flow path of the treating member 10 is designed by means of a device 52 for designing a treating member. More specifically, the device 52 for designing a treating member, as a first step, arranges the pieces of path shape/dimension information for carrying out the respective unit chemical operations according to the time series, sequentially from the inlet toward the outlet. Specifically, as shown in Fig. 6, the pieces of flow path shape/dimension information corresponding to the respective unit chemical operations (1) to (7) are read out, the arrangement designs for the respective flow paths on the treating member is carried out, and the respective flow paths are arranged in such a way that the flow paths are shifted away little by little from the rotation center along the radial direction. The piece of arrangement information is accumulated in a device 53 for recording a treating member structure.

Additionally, in the present step, because the locations along the radial direction from the center of the respective flow path elements are determined, based on the locations along the radial direction and the pieces of operation information needed for the respective flow path elements, there are acquired pieces of drive information including the rotation numbers and the like in carrying out the respective treatments. More specifically, in the present step, there can be acquired a piece of drive control information about the device with which the chemical treatment process is carried out. The piece of drive control information is accumulated in a device 60 for recording the piece of drive information about the treating member. Incidentally, the piece of drive information accumulated in the device 60 for recording the piece of drive control information about a treating member is transmitted to the user terminal 1.

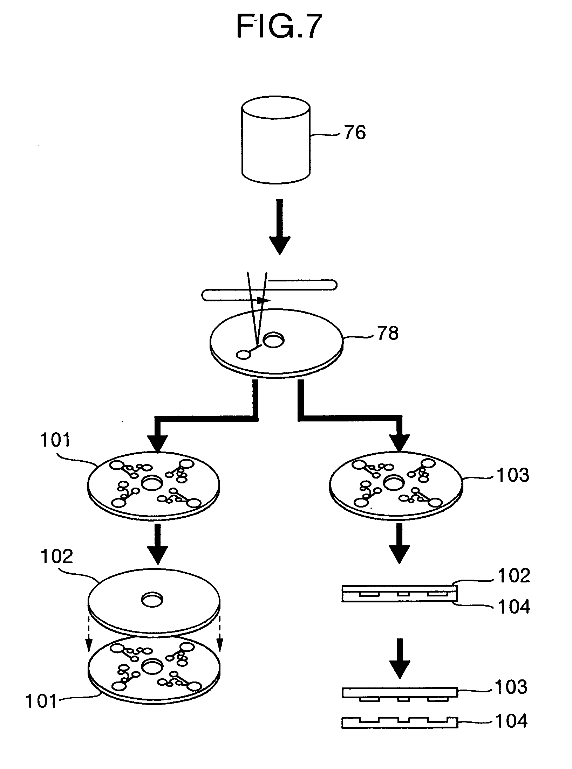

On the other hand, in step 4, the piece of design information about the treating member in the device 53 for recording a treating member structure is transmitted to a tool 70 for high speed processing of a treating member. The tool 70 for high speed processing of a treating member fabricates the treating member, based on the received piece of design information about the treating member, in a manner as shown in Fig. 7. Incidentally, the fabrication process of a treating member shown in Fig. 7 is an example, and the fabrication of a treating member based on the piece of design information about the treating member is not limited to the method shown in Fig. 7, but any other methods may be applied.

According to the method shown in Fig. 7, as a first step, based on the piece of flow path shape information, the surface 78 of a blank workpiece coated with a negative photosensitive resin is exposed to light in conformity with the flow path shape with the aid of a laser 76. Then, a corrosive liquid is applied onto the surface 78 of the blank workpiece to remove the exposed area. Consequently, a treating member 101 on which the desired flow path is formed can be fabricated, and is used in a condition such that a lid 102 is adhered thereto.

Alternatively, a stamper 103 may be made in such a way that the surface 78 of a blank workpiece coated with a positive photosensitive resin is exposed to light in conformity with the flow path shape with the aid of the laser 76, and a corrosive liquid is applied onto the surface to remove the resin while the flow path portion is left intact. In this case, a treating member 104 can be fabricated in such a way that a resin is poured onto the stamper 103, and after curing, the resin is released.

The treating member fabricated as described above is used in a device owned by the user side and shown in Fig. 3, and repeats rotation/halt according to the piece of drive control information. Incidentally, the piece of drive control information may be made to be arbitrarily alterable in pieceting on the user side. Hereby, chemical experiments based on altering various types of parameters can be easily carried out.

As described above, according to a system and a program for designing a treating member involved in the present invention, there can be acquired a piece of design information about a treating member desired by the user, and concurrently a piece of drive control information about the treating member fabricated based on the concerned piece of design information. Accordingly, on the user side, by merely presenting at least a desired treatment sequence, there can be obtained a piece of drive control information about the device to be used for the treating member capable of carrying out the treatment sequence concerned and for the treatment process using the treating member concerned.

### <Second best embodiment>

In the aforementioned first best embodiment, based on the piece of input information entered by the system user, the design of the treating member is carried out on the provision terminal side; however, the technical scope of the present invention is not limited to the first best embodiment.

In other words, as a second best embodiment applied with the present invention, the system for designing a treating member may be a system in which a piece of design information about the treating member 10 is acquired on the user terminal 1 belonging to the system user side, and the acquired piece of design information is output toward the provision terminal. In this case, a program for designing a treating member is installed in the user terminal 1, and steps 1 up to 3 shown in Fig. 4 are executed on the user terminal 1. In this connection, the program for designing a treating member may be arranged to be downloaded into the user terminal 1 after the system user have received certification from the provision terminal 2.

Additionally, the user terminal 1 outputs the piece of design information about the treating member acquired at step 3 toward the provision terminal. Hereby, on the provision terminal 2 side, the acquired piece of design information about the treating member is transmitted to the tool 70 for high speed processing of the treating member (step 4). Also in the present system, the tool 70 for high speed processing of the treating member can fabricate the treating member in a manner as shown in Fig. 7, based on the received piece of design information about the treating member.

In the present system, on the user terminal 1 on the system user side, the piece of design information about the treating member is acquired by use of the program for designing a treating member, and consequently, no piece of input information is needed to be transmitted from the user terminal 1 to the provision terminal 2. The piece of input information includes pieces of information which the system user does not want to disclose such as a piece of information about the unit chemical operations of the chemical treatment process desired by the user side. According to the present system, merely the piece of design information about the treating member is output from the system user to the provision terminal 2 side, and hence a piece of information which the system user does not want to disclose can be concealed from the provider side.

It should be further understood by those skilled in the art that although the foregoing description has been made on embodiments of the invention, the invention is not limited thereto and various changes and modifications may be made without departing from the spirit of the invention and the scope of the appended claims.

## Claims

1. A computer program embodied on a computer readable medium for designing a treating member (10), comprising:
a first code segment adapted to acquiring a piece of input information comprising a treatment sequence (ST1); and
a second code segment adapted to designing a flow path along which a sample moves, based on said piece of input information (ST2).

2. The computer program according to claim 1, wherein said piece of input information further comprises one or more pieces of information selected from the group consisting of a piece of physical property value information about said sample, a piece of treatment condition information about said sample, and a piece of liquid sample related information.

3. The computer program according to claim 1 or 2, wherein said flow path is designed by optimizing a piece of shape information, read out from a database in which the treatment sequence and the piece of shape information are stored to correlate with each other, based on a piece of information included in said piece of input information.

4. The computer program according to at least one of claims 1 to 3, further comprising a third segment adapted to outputting a piece of design information about the designed flow path.

5. The computer program according to at least one of claims 1 to 4, further comprising a fourth segment adapted to constructing a piece of drive control information about the designed treating member.

6. A system for providing a service to design a treating member (10), the system comprising the steps of:
acquiring a piece of design information about a flow path along which a sample moves, designed based on a piece of input information comprising a treatment sequence;
creating a piece of control information for controlling a treating-member fabrication unit (70) based on said piece of design information; and
outputting said piece of control information to said treating-member fabrication unit (70).

7. The system according to claim 6, wherein said piece of input information further comprises at least one piece of information selected from the group consisting of a piece of physical property value information about said sample, a piece of treatment condition information about said sample, and a piece of liquid sample related information.

8. The system according to claim 6 or 7, wherein said flow path is designed by optimizing a piece of shape information, read out from a database in which a treatment sequence and the piece of shape information are stored so as to correlate with each other, based on a piece of information included in said piece of input information.

9. The system according to at least one of claims 6 to 8, further comprising the step of constructing a piece of drive control information about the designed treating member (10), and outputting a piece of drive control information about the designed treating member (10) to a terminal to which has output said piece of design information.

10. A system for providing a service to design a treating member (10), comprising the steps of:
acquiring a piece of input information comprising a treatment sequence;
designing a flow path along which a sample moves based on said piece of input information;
creating a piece of control information for controlling a treating-member fabrication unit (70), based on a piece of design information about the designed flow path; and
outputting said piece of control information to said treating-member fabrication unit (70).

11. The system according to claim 10, wherein said piece of input information further comprises at least one piece of information selected from the group consisting of a piece of physical property value information about said sample, a piece of treatment condition information about said sample, and a piece of liquid sample related information.

12. The system according to claim 10 or 11, wherein said flow path is designed by optimizing a piece of
shape information, read out from a database in which a treatment sequence and the piece of shape information are stored so as to correlate with each other, based on a piece of information included in said piece of input information.
